# EUROPÄISCHE PATENTSCHRIFT

(11) **EP 0 664 116 B1**
(45) Veröffentlichungstag und Bekanntmachung des Hinweises auf die Patenterteilung: **02.09.1998**
(21) Anmeldenummer: 95100156.9
(22) Anmeldetag: 07.01.1995
(51) Int. Cl.: A61K 9/127

(54) **Verfahren zur Herstellung von Liposomen bzw. Proliposomen**
Process for preparing liposomes and/or proliposomes
Procédé pour la préparation de liposomes et/ou préliposomes

(30) Priorität: 20.01.1994 CH 174/94
(43) Veröffentlichungstag der Anmeldung: 26.07.1995
(73) Patentinhaber: F. HOFFMANN-LA ROCHE AG, 4002 Basel (CH)
(72) Erfinder: Ganter, Sabina, D-79365 Rheinhausen (DE); Völker, Karl Manfred, D-79100 Freiburg (DE)
(74) Vertreter: Kellenberger, Marcus, Dr.

(56) Entgegenhaltungen:
- EP-A- 0 499 299
- EP-A- 0 521 398
- WO-A-87/07502
- WO-A-94/28876
- W. GERHARTZ 'ULLMANN'S ENCYCLOPEDIA OF INDUSTRIAL CHEMISTRY , 5th edition, vol. B 2' 1988 , VCH VERLAGSGESELLSCHAFT MBH , WEINHEIM (DE) * Seite 5-20 - Seite 5-21, Absatz 2.2. * * Seite 5-36 - Seite 5-38, Absatz 2.6 *

## Beschreibung

Die Erfindung betrifft ein Verfahren zur Herstellung von Liposomen bzw. Proliposomen, wobei eine zur deren Herstellung geeignete Mischung in einer Rührwerks-Kugelmühle gemahlen wird.

Auf Grund der Fähigkeit der Liposomen in die Haut zu penetrieren, können sie unter anderem in der Kosmetik oder in der dermalen Therapie eingesetzt werden. Sie können mit Feuchtigkeit oder aber den verschiedensten Wirkstoffen, unter anderem mit Vitaminen beladen werden. Dank der amphiphilen Eigenschaften der Liposomen werden diese Wirkstoffe weit in die Haut transportiert, und somit wird die Abgabe dieser Wirkstoffe an der gewünschten Stelle gewährleistet.

Verschiedene Methoden zur Herstellung von Liposomen und Proliposomen sind bekannt. Allerdings sind die meisten dieser Methoden nur im Labormaßstab geeignet, so zum Beispiel die Filmmethode. Solche Methoden sind dem Fachmann bekannt und beispielsweise in *Liposome Technology; Gregoriadis G., (ed.), Vol. I-III (1993)* beschrieben. Zur Herstellung von Liposomen in grösserem Maßstab eignen sich beispielsweise die Rotor-Stator-Homogenisation oder die Flüssigkeitshochdruck-Homogenisation. Obwohl die Flüssigkeitshochdruck-Homogenisatoren, wozu z.B. Flüssigkeitsstrahlen-Hochdruckhomogenisatoren (Microfluidizer) oder Flüssigkeitsspaltdüsen-Hochdruckhomogenisatoren gehören, sich sehr gut für die Herstellung von Liposomen eignen, haben sie den Nachteil, dass mit ihnen nur flüssige Materialien verarbeitet werden können. Sie eignen sich nicht für sehr konzentrierte und viskose Zubereitungen. Zudem befriedigt die Reproduzierbarkeit der Teilchengrösse vor allem bei der Herstellung von Liposomen mit Flüssigkeitsspaltdüsen-Hochdruckhomogenisatoren nicht immer. Ein weiterer Nachteil der genannten Herstellungsmethoden ist die Tatsache, dass hohe Energien auf die Teilchen einwirken.

Es stellte sich nun die Aufgabe, ein Verfahren zur Herstellung von Liposomen und Proliposomen zu finden, bei welchem schonend und ohne grosse Verdünnung gearbeitet werden kann. Das Verfahren sollte ermöglichen, auf schonende Weise Liposomen bzw. Proliposomen mit einer regelmässigen Struktur herzustellen, in welchen gegebenenfalls zugegebene Wirkstoffe gleichmässig verteilt sind. Ausserdem sollte das Verfahren auch ohne Weiteres grosstechnisch anwendbar sein.

Es wurde nun gefunden, dass Liposomen und/oder Proliposomen auf schonende Weise durch Mahlen einer zur Herstellung von Liposomen und/oder Proliposomen geeigneten Mischung in einer Rührwerks-Kugelmühle hergestellt werden können.

Eine Rührwerks-Kugelmuhle ist eine besondere Form der Kugelmühle. Die Homogenisierung und Verkleinerung wird in dieser Mühle durch Kräfte bewirkt, die durch das Rührwerk auf die freibeweglichen Kugeln, vorzugsweise aus Glas, und von dort auf das Mahlgut übertragen werden.

Das erfindungsgemässe Verfahren ist sehr schonend und kann auch grosstechnisch angewendet werden. Es eignet sich gleichermassen zur Herstellung von Proliposomen, zur Herstellung von Liposomen aus Proliposomen wie auch zur direkten Herstellung von Liposomen. Durch die Wahl der Homogenisierungsbedingungen (z. B. Rotor-Umfangsgeschwindigkeit, Mahldauer, Kugelgrösse, Füllgrad, Temperatur) können Grösse und Lamellarität der Proliposomen bzw. Grösse der Liposomen gezielt variiert werden.

Durch das erfindungsgemässe Verfahren können sowohl unbeladene als auch mit einer oder mehreren lipophilen und/oder hydrophilen Substanzen beladene Proliposomen bzw. Liposomen hergestellt werden.

In Mischungen, welche zur Herstellung von Liposomen bzw. Proliposomen verwendet werden können, werden Lecithine unterschiedlicher Herkunft wie beispielsweise Ei-Lecithin oder Soja-Lecithin verwendet; solche Lecithine sind im Handel erhältlich.

Als Lösungsvermittler können polare organische Lösungsmittel, beispielsweise Alkohole wie Ethanol, iso-Propanol und dergleichen eingesetzt werden.

Als einzubauende Verbindungen kommen lipophile oder hydrophile Substanzen, wie lipophile oder hydrophile Vitamine, Pharmazeutika sowie Aromen und Riechstoffe in Frage. Es sind dies beispielsweise die fettlöslichen Vitamine und Vitamin-Derivate (A, K und E) , deren Vorstufen (β-Carotin), Carotinoide oder fettlösliche Pharmazeutika wie Diazepam, Amphotericin B, Econazol, Corticoide und dergleichen. Als hydrophile Verbindungen kommen beispielsweise die wasserlöslichen Vitamine (B-Komplex oder Vitamin C) oder hydropbile Pharmazeutika wie Heparin, Cisplatin und dergleichen, in Frage. Beispiele fettlöslicher Aromen und Riechstoffe sind Citrusöle, Pfefferminzöl und dergleichen. Als hydrophile Aromen und Riechstoffe können beispielsweise Vanille-Extrakt, Kakao-Aroma oder Tabak-Aromen eingebaut werden.

Durch das erfindungsgemässe Verfahren werden die zugefügten lipophilen bzw. hydrophilen Verbindungen auf sehr schonende Weise in die Liposomen bzw. Proliposomen eingearbeitet. Es wird eine sehr gute Durchmischung und somit eine homogene Verteilung der zugefügten Verbindungen im Bilayer gewährleistet.

Bei der Herstellung von Proliposomen kann bei dem erfindungsgemässen Verfahren im Gegensatz zu den herkömmlichen Methoden mit sehr konzentrierten und viskosen Lecithin-Mischungen gearbeitet werden.

Eine für die Herstellung von Proliposomen mittels einer Rührwerks-Kugelmühle geeignete Mischung enthält
50-90% Lecithin
15-50% Lösungsvermittler
0-10% Wasser
0-20% einzubauende hydrophile und/oder lipophile Verbindungen.

Die Herstellung von Proliposomen durch das erfindungsgemässe Verfahren erfolgt in typischer Weise dadurch, dass eine Mischung, die nach oben angegebener Rezeptur zubereitet worden ist, in einer Rührwerks-Kugelmühle gemahlen wird. Dabei kann - je nach gewünschter Struktur - die Rührwerks-Kugelmühle zwischen 50 und 90 % des Volumens mit handelsüblichen Kügelchen von 0.2 bis 6 mm, vorzugsweise Glaskügelchen von 0.3 bis 1.5 mm Durchmesser, beschickt werden. Durch die Mahlgeschwindigkeit, die Mahldauer sowie die Temperatur kann die Struktur des Produktes beeinflusst werden. Die Rotor-Umfangsgeschwindigkeit beträgt vorzugsweise 5-15 m/s.

Proliposomale Formulierungen, die durch das Verfahren hergestellt werden, zeichnen sich durch eine sehr regelmässige, lange lamellare Struktur aus. Sie enthalten sehr hohe Lecithin-Konzentrationen, und gegebenenfalls zugegebene Wirkstoffe sind sehr homogen verteilt.

Die Proliposomen, die durch das erfindungsgemässe Verfahren hergestellt werden, sind wegen ihrer hohen Konzentration und ihrem relativ hohen Gehalt an Alkohol sehr gut konservierbar.

Die oben beschriebenen Proliposomen können nach Zugabe von Wasser auf bekannte Weise, wie beispielsweise durch Homogenisation mit einem Flüssigkeitsstrahlen-Hochdruckhomogenisator, in Liposomen übergeführt werden. Dabei ist bemerkenswert, dass die Liposomen aus erfindungsgemäss hergestellten proliposomalen Zubereitungen in der Regel kleiner sind als Liposomen, welche direkt hergestellt werden.

Aus den oben beschriebenen Proliposomen können die Liposomen nach Zugabe von Wasser auch erfindungsgemäss durch Mahlen der proliposomalen Zubereitung in einer Rührwerks-Kugelmühle hergestellt werden. In typischer Weise wird dazu die proliposomale Zubereitung mit Wasser versetzt bis der Wassergehalt zwischen 35 und 95 % beträgt und danach in der Rührwerks-Kugelmühle gemahlen.

Wie bereits erwähnt, können durch Mahlen von geeigneten Mischungen in der Rührwerks-Kugelmühle erfindungsgemäss auch direkt Liposomen hergestellt werden. Eine für die Herstellung von Liposomen mittels einer Rührwerks-Kugelmühle geeignete Mischung enthält
1-30% Lecithin
1-15% Lösungsvermittler
35-95% Wasser
0-5% einzubauende hydrophile und/oder lipophile Verbindungen.

Die Rührwerks-Kugelmühle wird dabei zwischen 50 und 90 % des Volumens mit handelsüblichen Kügelchen von 0.2 bis 6 mm, vorzugsweise von 0.3 bis 1.5 mm Durchmesser, beschickt. Durch die Mahlgeschwindigkeit sowie die Mahldauer kann auch in diesem Fall die Struktur des Produktes beeinflusst werden. Die Rotor-Umfangsgeschwindigkeit beträgt 5-15 m/s. Auf diese Weise können gezielt grosse und sehr homogene Liposomen (1 - 5 µm) hergestellt werden.

Die nachstehenden Beispiele sollen die Erfindung weiter erläutern.

### Beispiel 1

### Herstellung einer proliposomalen Lösung

- Rezeptur :: 120 g Lecithin (= 60 % G/G)
24 g Tocopherol-Acetat (= 12 % G/G)
42 g Ethanol (= 21 % G/G)
ad 200 g Aqua dest. (= 7 % G/G)
Eine Mischung von Lecithin, Ethanol und dl-α-Tocopherol wurde in einer Rührwerks-Kugelmühle 20 Min. unter Kühlung bei 15 m/s Rotor-Umfangsgeschwindigkeit gemahlen. Hierbei wurden Glasperlen von 1mm Durchmesser verwendet. Es entstanden sehr lange lamellare Strukturen. Die Formulierung war dickflüssig aber noch fliessfähig.

### Beispiel 2

### Herstellung von Liposomen

- Rezeptur :: 20 g Soja-Lecithin (= 10 % G/G)
7 g Ethanol (= 3,5 % G/G)
2 g Tocopherol-Acetat (= 1 % G/G)
ad 200 g Aqua dest. (= 85,5 % G/G)
Die Mischung wird zunächst 5 Min. hydratisiert und anschliessend unter Kühlung 20 Min. in der Rührwerks-Kugelmühle bei 10 m/s Rotor-Umfangsgeschwindigkeit gemahlen. Hierbei wurden Glasperlen von 0,3 mm Durchmesser verwendet. Es entstanden Liposomen mit einem Durchmesser von 1 - 2 µm.

### Beispiel 3

### Herstellung von Liposomen

- Rezeptur:: 20 g Soja-Lecithin (= 10% G/G)
7 g Ethanol (=3,5% G/G)
8 g Tocopherol-Acetat (= 4% G/G)
ad 200 g Aqua dest. (= 82,5% G/G)
Die Mischung wurde zunächst 5 Min. hydratisiert und anschliessend unter Kühlung 60 Min. in der Rührwerks-Kugelmühle bei 10 m/s Rotor-Umfangsgeschwindigkeit gemahlen. Es wurden Glasperlen von 0.3 mm Durchmesser verwendet. Es entstanden multilamellare Liposomen mit einer Grösse von 2-5 µm.

### Beispiel 4

### Herstellung von Liposomen aus Proliposomen

- Rezeptur:: 31 g Proliposomen aus Beispiel 1
(60% Lecithin, 12% Tocopherol-Acetat, 21%
Ethanol, 7% Aqua dest.)
ad 200 g Aqua dest.
Die Mischung wurde zunächst 5 Min. auf dem Magnetrührer gerührt und anschliessend in der Rührwerks-Kugelmühle 15 Min. bei 10 m/s Rotor-Umfangsgeschwindigkeit mit 0.5 mm Glasperlen gemahlen. Es entstanden unilamellare Liposomen von 50-100 nm Durchmesser folgender Endzusammensetzung:
18,6 g Sojalecithin (= 9,3% G/G)
6,51 g Ethanol (= 3,26% G/G)
3,72 g Tocopherol-Acetat (= 1,86% G/G)
171,17 g Aqua dest. (= 85,6% G/G)

## Patentansprüche

1. Verfahren zur Herstellung von Liposomen oder Proliposomen, dadurch gekennzeichnet, dass eine für die Herstellung von Liposomen bzw. Proliposomen geeignete Mischung in einer Rührwerks-Kugelmühle gemahlen wird.

2. Verfahren zur Herstellung von Liposomen aus Proliposomen, dadurch gekennzeichnet, dass eine proliposomale Zubereitung in einer Rührwerks-Kugelmühle unter Zusatz vor Wasser gemahlen wird.

3. Verfahren gemäss Anspruch 1 , dadurch gekennzeichnet, dass die für die Herstellung von Proliposomen geeignete Mischung weniger als 10% Wasser enthält.

4. Verfahren gemäss einem der Ansprüche 1 bis 3, dadurch gekennzeichnet, dass die Rotor-Umfangsgeschwindigkeit der Rührwerks-Kugelmühle 5-15 m/s beträgt.

5. Verfahren gemäss einem der Ansprüche 1 bis 4, dadurch gekennzeichnet, dass die Rührwerks-Kugelmühle mit Glaskügelchen von 0.3 bis 1.5 mm Durchmesser beschickt wird.

6. Verwendung einer Rührwerks-Kugelmühle zur Herstellung von Liposomen oder Proliposomen, gemäss einem der Ansprüche 1 bis 5.

7. Verwendung einer Rührwerks-Kugelmühle zur Herstellung von Liposomen aus Proliposomen, gemäss Anspruch 2, 4 oder 5.

## Claims

1. A process for the manufacture of liposomes or proliposomes, characterized in that a mixture suitable for the manufacture of liposomes or proliposomes is milled in an agitator-ball mill.

2. A process for the manufacture of liposomes from proliposomes, characterized in that a proliposomal composition is milled in an agitator-ball mill with the addition of water.

3. A process according to claim 1, characterized in that the mixture suitable for the manufacture of proliposomes contains less than 10% water.

4. A process according to any one of claims 1 to 3, characterized in that the rotor circumferential speed of the agitator-ball mill is 5-15 m/s.

5. A process according to any one of claims 1 to 4, characterized in that the agitator-ball mill is charged with glass beads of diameter 0.3 to 1.5 mm.

6. The use of an agitator-ball mill for the manufacture of liposomes or proliposomes according to any one of claims 1 to 5.

7. The use of an agitator-ball mill for the manufacture of liposomes from proliposomes according to claim 2, 4 or 5.

## Revendications

1. Procédé pour la préparation des liposomes ou des proliposomes, caractérisé en ce que l'on broye un mélange approprié pour la préparation des liposomes ou des proliposomes dans un broyeur agitateur à billes.

2. Procédé pour la préparation des liposomes à partir des proliposomes, caractérisé en ce que l'on broye une préparation proliposomale dans un broyeur agitateur à billes en additionnant de l'eau.

3. Procédé selon la revendication 1, caractérisé en ce que le mélange approprié pour la préparation des proliposomes contient moins de 10% d'eau.

4. Procédé selon l'une des revendications 1 à 3, caractérisé en ce que la vitesse circonférentielle du rotor du broyeur agitateur à billes est comprise entre 5 et 15 m/s.

5. Procédé selon l'une des revendictions 1 à 4, caractérisé en ce que le broyeur agitateur à billes est chargé de petites billes de verre ayant un diamètre compris entre 0,3 et 1,5 mm.

6. Utilisation d'un broyeur agitateur à billes pour la préparation des liposomes ou des proliposomes selon l'une des revendications 1 à 5.

7. Utilisation d'un broyeur agitateur à billes pour la préparation des liposomes à partir des proliposomes selon l'une des revendications 2, 4 ou 5.
